# EUROPEAN PATENT APPLICATION

(11) **EP 4 714 954 A1**
(43) Date of publication of application: **25.03.2026**
(21) Application number: 24201424.9
(22) Date of filing: 19.09.2024
(51) Int. Cl.: C07D 487/06, C07D 487/16, C07D 487/22, H01M 4/60

(54) **FUSED POLYCYCLIC COMPOUNDS AS ELECTRODE ACTIVE MATERIALS FOR AQUEOUS AND NON-AQUEOUS SECONDARY BATTERIES**

(71) Applicant: Kemijski Institut, 1000 Ljubljana (SI)
(72) Inventor: MENART, Svit, 1000 Ljubljana (SI); DOMINKO, Robert, 1000 Ljubljana (SI); PIRNAT, Klemen, 1000 Ljubljana (SI)
(74) Representative: Weickmann & Weickmann PartmbB

(57) **Abstract**

The present invention relates to fused polycyclic compounds, methods of preparing said compounds, their use as electrode active material, electrodes comprising said compounds, a method of preparing said electrodes, use of said electrodes in an aqueous or non-aqueous secondary battery and a secondary battery comprising said electrode.

## Description

The present invention relates to fused polycyclic compounds, methods of preparing said compounds, their use as electrode active material, electrodes comprising said compounds, a method of preparing said electrodes, use of said electrodes in an aqueous or non-aqueous secondary battery and a secondary battery comprising said electrode.

The large-scale deployment of battery technology is intensifying the search for more sustainable and less scarce materials. Currently, state-of-the-art batteries are based on lithium-ion technology, where the cathodes comprise transition metal ceramics such as lithium cobalt oxide (LCO), lithium iron phosphate (LFP), and nickel manganese cobalt oxide (NMC). Resource scarcity and uneven distribution of the elements used in conventional batteries can present constraints for their further adoption.

In recent years, a more sustainable alternative to the presently used transition metal ceramics has emerged in the form of organic cathode materials, which can be made out of abundant raw materials with a lower carbon footprint.

In contrast to the rigid lattices of inorganic materials, organic materials possess flexibility and can swell enabling their use in multivalent batteries such as zinc, magnesium and aluminum. These mentioned elements are not only much more abundant than lithium, but also possess higher volumetric capacities enabling increased energy densities of cells. Among them, zinc metal shows the highest redox potential and high hydrogen evolution overpotential rendering it suitable for the use with aqueous electrolytes, which increases the safety and reduces the cost of the cells.

Even though different organic cathode materials are already available, many available materials suffer from difficult synthesis, low energy densities and/or short cycle life. Thus, the task of the present invention is to provide compounds for electrode active materials available through facile synthesis, which possess high energy densities and enable long cycle life. Preferably, these compounds should work both with non-aqueous and with aqueous electrolytes. Among the different types of organic cathode materials, it has been found that materials based on the combination of pyrazine and quinone motifs possess some of the best characteristics.

### Detailed description of the invention

To overcome the limitations of presently available compounds used as organic cathode materials, the present inventors have developed compounds based on a novel combination of pyrazine and quinone redox active motifs. These compounds can be employed as electrode active material in aqueous and non-aqueous batteries, where the electrically conductive material of the anode preferably comprises at least one of Li, Zn, Mg, Al, K and Na.

Thus, in a first aspect, the present invention relates to a fused polycyclic compound represented by any one of the general formulas (I) to (III) 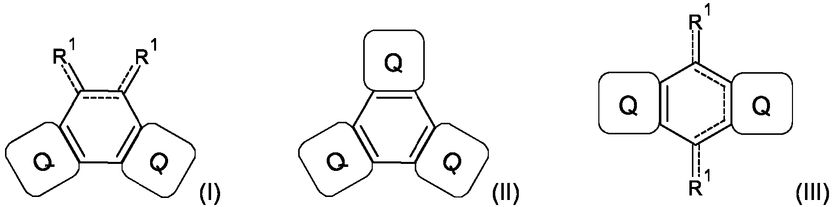 with Q being represented by the general formula (IV) 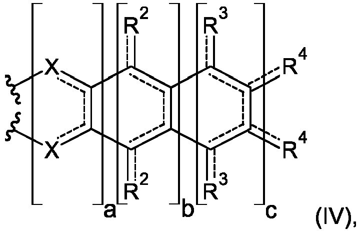 wherein
- X: is 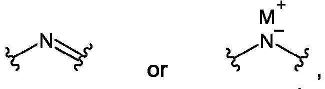
- R¹: is 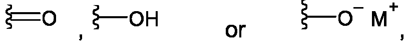
- R²: is independently selected from or a linear or 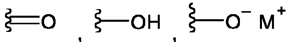 branched, preferably linear, alkoxy group with 1-6 C atoms, preferably with 1-3 C atoms,
- R³: is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,
- R⁴: is independently selected from a hydrogen 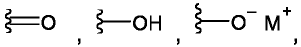 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
- M⁺: represents cations independently selected from a group represented by protons, Li⁺, Na⁺, K⁺, ½ Mg²⁺, ½ Ca²⁺ and ½ Zn²⁺,
- a: is an integer of 1-2,
- b: is an integer of 0-2, and an integer of 1-2 if the fused polycyclic compound corresponds to formula (II),
- c: is an integer of 0-1,

wherein the sum of integers a, b and c is 2-4, and
bonds that are represented by a solid line and a dashed line indicate a single bond or a double bond.

For the purpose of the present application, a notation of the general form ½ M²⁺, such as ½ Mg²⁺, ½ Ca²⁺ or ½ Zn²⁺, indicates that M²⁺ ions, such as Mg²⁺, Ca²⁺ or Zn²⁺ ions, are present in an amount that ensures that they contribute a net charge of +1 for each instance of M⁺ or ½ M²⁺ in the respective formula.

Moreover, in each instance within each formula of the present application in which a C atom forms multiple bonds indicated with a solid and a dashed line, it is preferred that one of the bonds is a double bond and all other bonds are single bonds. Accordingly, for multiple subsequent bonds indicated with a solid and a dashed line, it is preferred that they form a conjugated system of alternating single and double bonds.

If R⁴ is 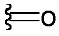 , it is bound to the outermost ring of Q (as seen from the center of the fused polycyclic compound) via a double bond regardless of the values of a, b and c. In this case, the C atoms bound to R⁴ form one double bond to R⁴ and a single bond each to the adjacent C atoms within the ring. Preferably, R⁴ is a single-bond substituent.

Formulas with different but chemically equivalent notations to the above formulas are herewith understood to be included in the above formulas. For example, compounds of the present invention may be represented by formulas corresponding to the above formulas, wherein conjugated systems are written in an alternative but chemically equivalent manner.

Preferably, Q is identical within one compound, e.g. within general formula (i), within general formula (II) and/or within general formula (III).

In a preferred embodiment of the invention, Q in general formulas (I), (II) and/or (III) is represented by formula (IVa) 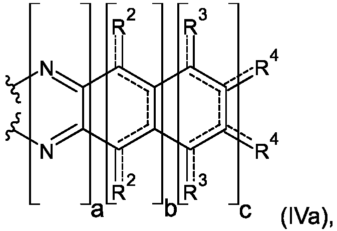 with R²-R⁴ and a-c being defined as described above.

For R¹, R² and R³, it is preferred that two substituents bound to a same ring in a compound of the invention are identical. For R⁴, the two substituents in Q may be identical or different. If R⁴ is 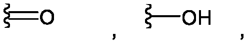 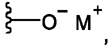 a hydrogen atom, halogen atom a or a C₁₋₆-alkyl or C₁₋₆-alkoxy group, it is preferred that the two R⁴ in Q are identical. If one R⁴ is a nitro or C₁₋₆-carboxy group, it is preferred that the other R⁴ in Q is a hydrogen atom.

In a particularly preferred embodiment of the invention, the fused polycyclic compound is represented by general formula (I), and
a = 2, b = 0 and c = 1, or
a = 1, b = 1 and c = 0, or
a = 1, b = 2 and c = 1, or
a = 2, b = 0 and c = 0,
preferably a = 2, b = 0 and c = 1 or a = 1, b = 2 and c = 1.

In an alternative preferred embodiment, the fused polycyclic compound is represented by general formula (II), and
a = 1, b = 1 and c = 0, or
a = 1, b = 2 and c = 1,
preferably a = 1, b = 2 and c = 1.

In an alternative preferred embodiment, the fused polycyclic compound is represented by general formula (III), and
a = 2, b = 0 and c = 1, or
a = 1, b = 1 and c = 0, or
a = 1, b = 2 and c = 1, or
a = 2, b = 0 and c = 0.

Preferably, the fused polycyclic compound of the present invention is represented by any one of the following formulas:

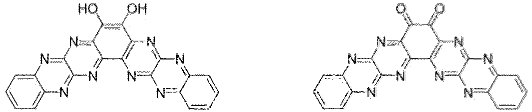

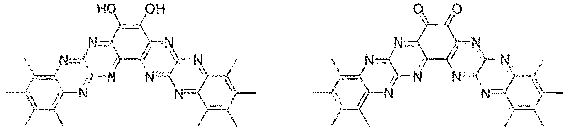

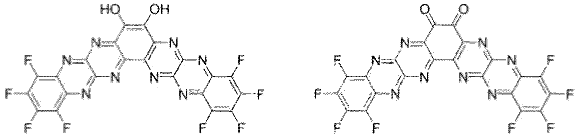

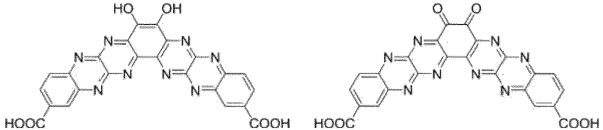

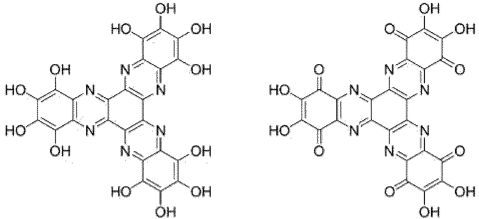

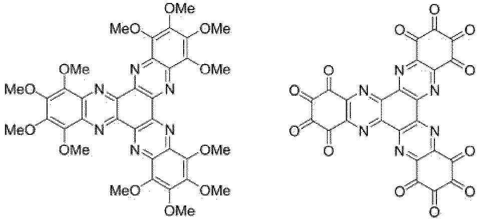

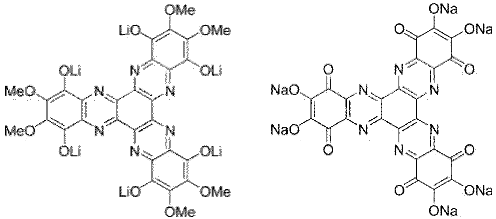

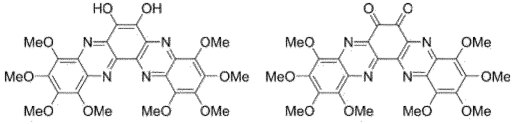

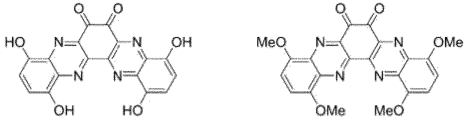

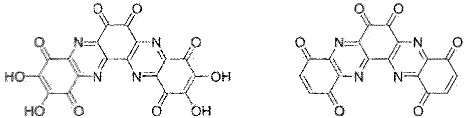

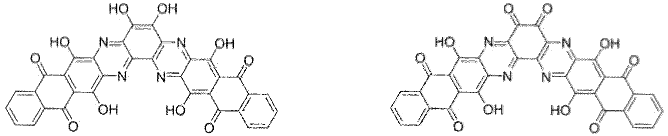

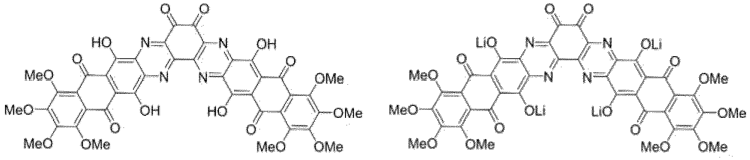

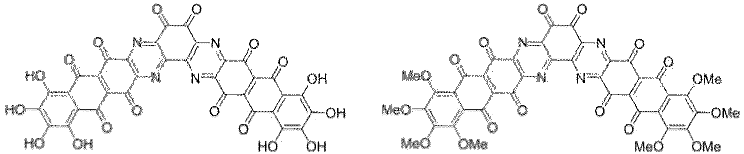

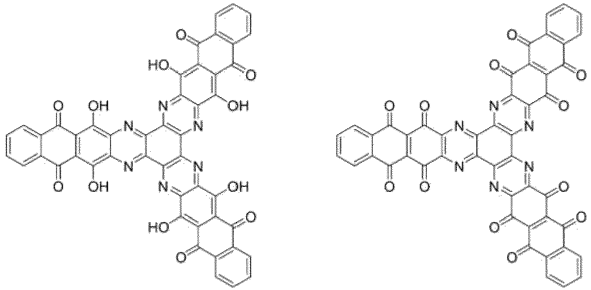

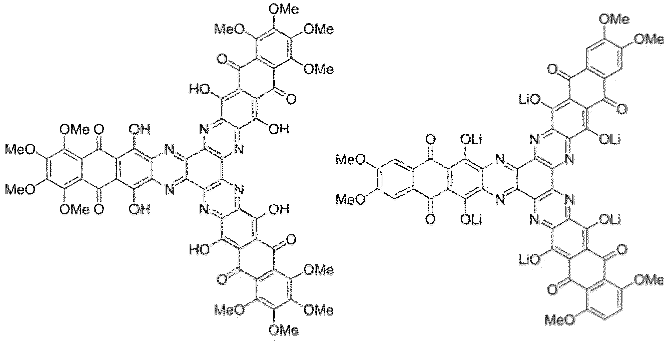

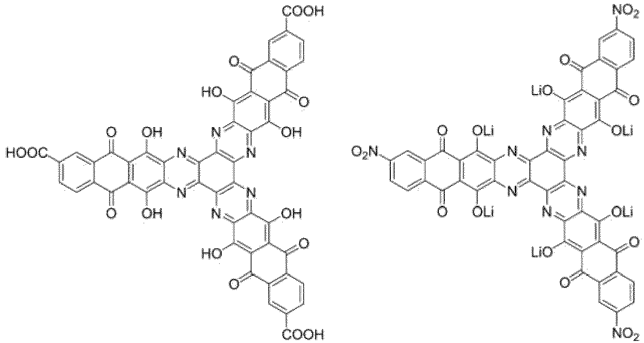

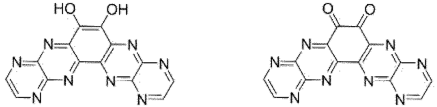

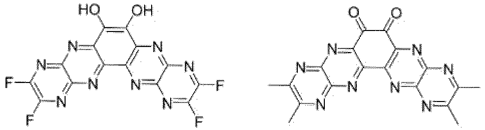

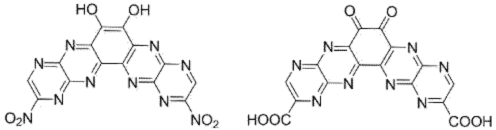

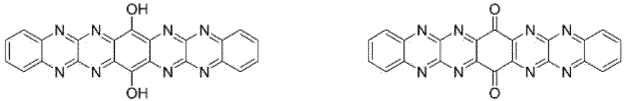

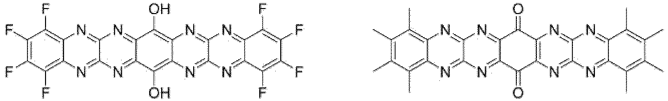

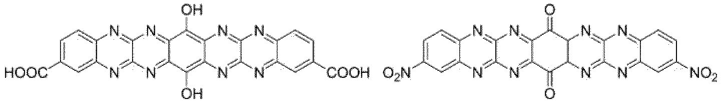

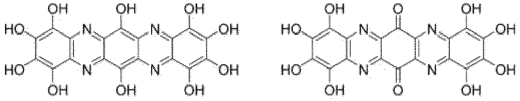

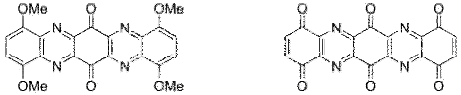

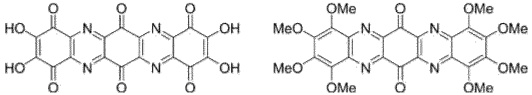

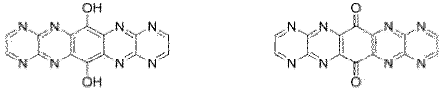

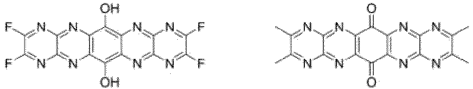

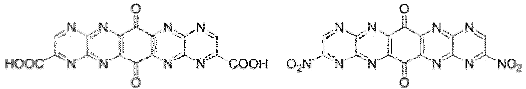

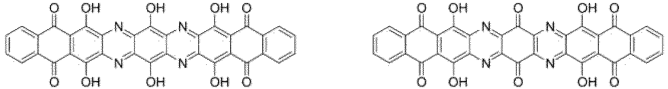

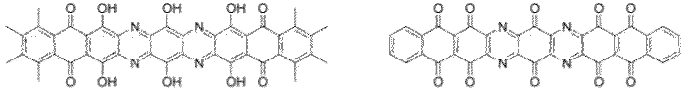

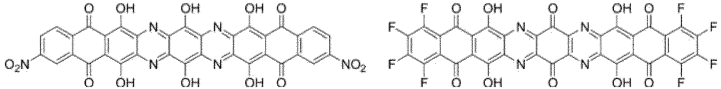

In particularly preferred embodiments of the present invention, the fused polycyclic compound is represented by any one of the following formulas:

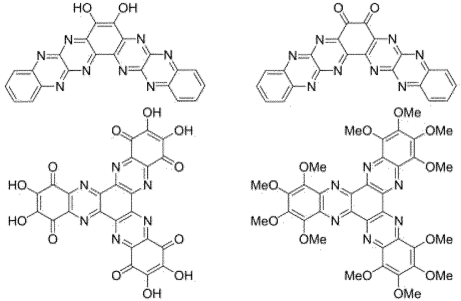

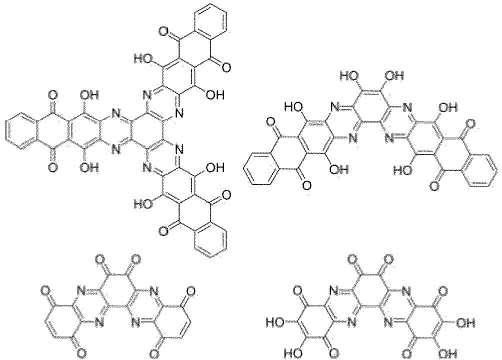

It has surprisingly been found that the fused polycyclic compounds of the invention are easy to synthesize and show advantageous characteristics when used as electrode active materials in both non-aqueous and aqueous batteries. In contrast to other known organic cathode materials, the compounds of the invention show good stability in both aqueous and non-aqueous electrolytes with favorable energy densities.

In another aspect, the present invention provides a method of preparing a fused polycyclic compound of the invention, wherein the method comprises reacting a compound of the general formula (V) 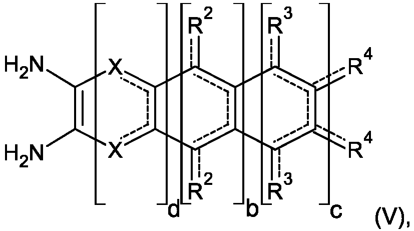 wherein
- X: is 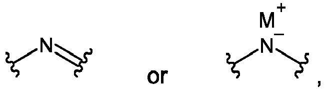
- R²: is independently selected from or a linear or 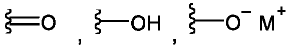 branched, preferably linear, alkoxy group with 1-6 C atoms, preferably with 1-3 C atoms,
- R³: is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,
- R⁴: is independently selected from a hydrogen 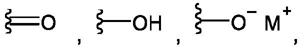 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
- M⁺: represents cations independently selected from a group represented by protons, Li⁺, Na⁺, K⁺, ½ Mg²⁺, ½ Ca²⁺ and ½ Zn²⁺,
- b: is an integer of 0-2, and an integer of 1-2 if the fused polycyclic compound corresponds to formula (II),
- c: is an integer of 0-1,
- d: is an integer of 0-1,

wherein the sum of integers b, c and d is 1-3, and
bonds that are represented by a solid line and a dashed line indicate a single bond or a double bond,
with a compound selected from

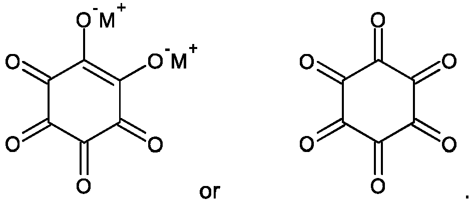

Preferably, the reaction is carried out in a solvent, preferably a deoxygenated solvent, wherein the solvent is preferably selected from glacial acetic acid, Methanol, ethanol, water, and mixtures thereof.

In a preferred embodiment of the method of the invention, a catalyst is added to the reaction, wherein the catalyst is preferably selected from 2-iodoxybenzoic acid, Zirconium(IV) chloride, sulfamic acid and ammonium chloride.

It is further preferred that the reaction of the method of the invention is conducted at a temperature of 110-130°C, preferably of 120°C, preferably for 8-72 h, more preferably for 16-48 h.

Moreover, in a preferred embodiment of the method of the invention, one or more of substituents R², R³ and R⁴ in formula (V) are protected by protecting groups. In this case, the method preferably further comprises a step of deprotecting said substituents with a deprotecting agent. Suitable protecting groups and deprotecting procedures are known to a person skilled in the art.

In a further preferred embodiment, the method of the invention further comprises an oxidation or reduction step, preferably after the reaction described above.

Preferably, the method of the invention further comprises purifying the product of the reaction. Suitable purification methods are known to the person skilled in the art. It is preferred that the product of the reaction is initially filtered and washed with a solvent, preferably with glacial acetic acid, acetone, ethanol, water, dichloromethane and/or dimethyl formamide. Next, the product of the reaction is preferably additionally purified by Soxhlet extraction with ethanol. In a final preferred step of the method of the invention, the product of the reaction is further dried at a temperature of 70-90°C, preferably of 80°C, for 3-24 hours, preferably overnight.

In one preferred embodiment, the method of the invention comprises the reaction of an o-diaminoquinoxaline compound with sodium rhodizonate according to the following reaction scheme: 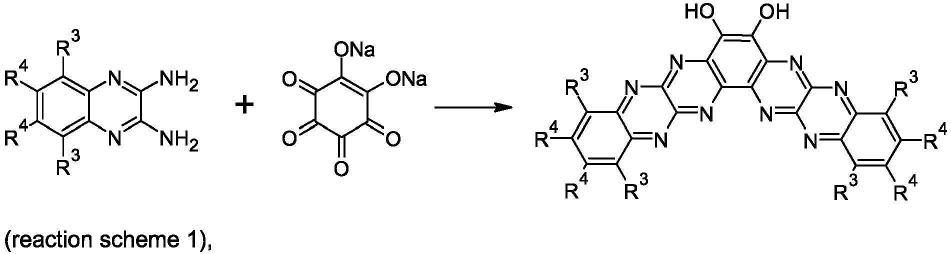 wherein R³ and R⁴ are defined as above.

Preferably, the reaction of reaction scheme 1 is carried out in a deoxygenated solvent, e.g. glacial acetic acid, with or without addition of a catalyst such as 2-iodobenzoic acid.

In another preferred embodiment, the method of the invention comprises a reaction according to the following reaction scheme: 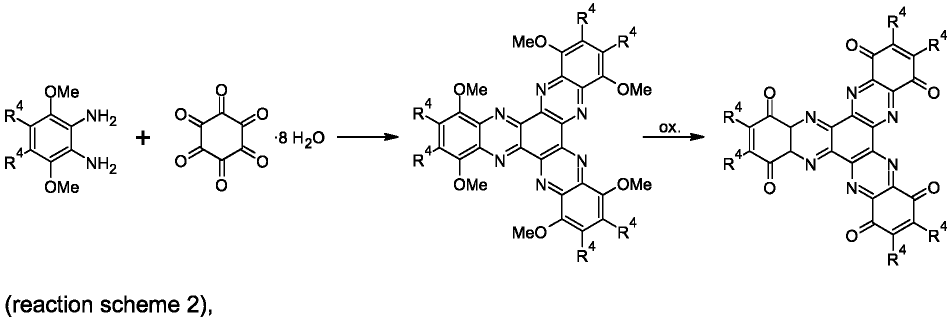 wherein R⁴ is defined as above.

In a first step of the reaction of reaction scheme 2, a 1,2-diamino-3,6-dimethoxybenzene derivative is reacted with hexaketocyclohexane octahydrate (HK) in deoxygenated solvent (e.g., glacial acetic acid) with or without the addition of catalyst (e.g., 2-iodoxybenzoic acid). In a second step, the methoxy groups are deprotected with a deprotecting agent (eg., pyridine hydrochloride) and the product is oxidized on air to yield the final compound.

In another preferred embodiment, the method of the invention comprises a reaction of a 2,3-diamino-1,4-dihydroxy-9,10-anthracenedione derivative with sodium rhodizonate according to the following reaction scheme: 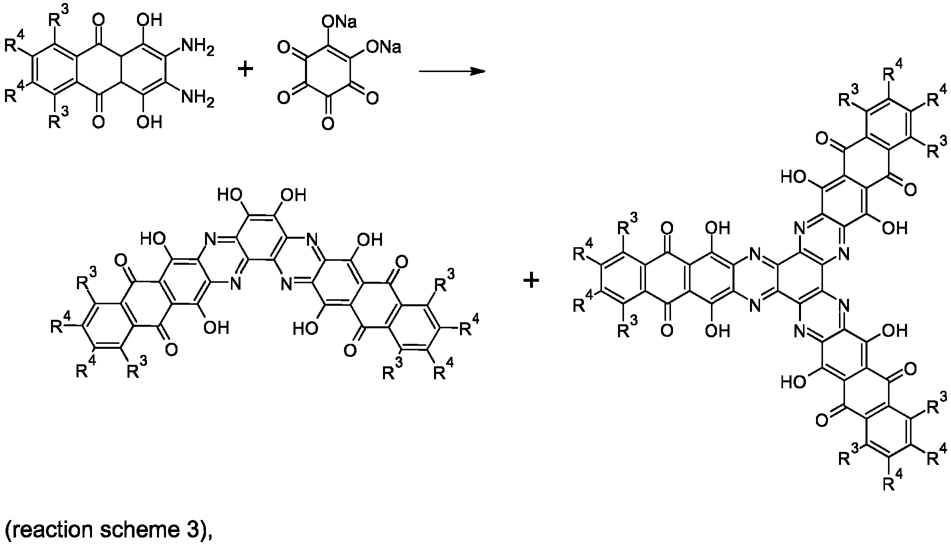 wherein R³ and R⁴ are defined as above.

As indicated, the reaction of reaction scheme 3 preferably yields a mixture of two compounds which are preferred embodiments of the compounds of general formulas (I) and (II). Preferably, the reaction of reaction scheme 3 is carried out in a deoxygenated solvent, e.g. glacial acetic acid, with or without addition of a catalyst such as 2-iodobenzoic acid.

In another aspect, the present invention relates to the use of the fused polycyclic compound of the invention as an electrode active material for an aqueous or non-aqueous secondary battery. Preferably, the compound of the invention is used as a cathode active material.

Furthermore, the present invention relates to an electrode for an aqueous and/or non-aqueous secondary battery, comprising
(i) an electrode active material comprising at least one fused polycyclic compound of the invention,
   and further comprising at least one of
(ii) a binder, and/or
(iii) a conductive material.

Preferably, the electrode of the invention comprises the electrode active material (i), the binder (ii) and the conductive material (iii).

The electrode of the invention is preferably a cathode.

Preferably, the electrode active material (i) is present in the electrode in an amount of 40-95 wt.%, preferably 50-80 wt.%, with respect to the total weight of the electrode.

The binder (ii) is preferably selected from any suitable binder known in the art, more preferably from polyvinylidene fluoride (PVDF), polytetrafluoroethylene, polyacrylic acid, sodium carboxymethyl cellulose, styrene butadiene rubber, and combinations thereof. If a binder (ii) is present in the electrode, it is preferably present in an amount of 2-20 wt.%, preferably 5-15 wt.%, with respect to the total weight of the electrode.

The conductive material (iii) is preferably a carbon-based conductive material, more preferably selected from graphite, carbon black, such as acetylene black, Ketjenblack, or Printex XE2, amorphous carbon materials, such as carbon materials prepared by forming amorphous carbon on the surface, fibrous carbon, such as vapor-grown carbon fiber and carbon fiber prepared by carbonization after spinning of pitch, carbon nanotubes, such as multi-walled or single-walled carbon nanotubes, and combinations thereof. If a conductive material (iii) is present in the electrode, it is preferably present in an amount of 2-30 wt.%, preferably 5-20 wt.%, with respect to the total weight of the electrode.

In a particularly preferred embodiment, the electrode of the invention comprises around 60 wt.% of electrode active material (i), around 10 wt.% of binder (ii) and around 30 wt.% of conductive material (iii). In an even more preferred embodiment, the electrode of the invention comprises 60 wt.% of electrode active material (i), 10 wt.% of binder (ii) and 30 wt.% of conductive material (iii).

In certain preferred embodiments, the electrode of the invention is formed on at least one surface of an electrode current collector, preferably a cathode current collector. Preferably, the thickness of the electrode on the surface of the current collector is in the range of 1 µm to 200 µm. The current collector, which is preferably a cathode current collector, is preferably a foil, mesh, perforated metal or expanded metal. More preferably, the current collector is made of a metal or metal alloy, preferably of aluminum, stainless steel, nickel, titanium or alloys thereof.

In another preferred embodiment, the electrode of the invention may also be a self-standing electrode.

A further aspect of the present invention relates to a method of preparing an electrode of the invention which is preferably a cathode, the method comprising the steps:
a) preparing a mixture comprising the electrode active material (i), at least one of a binder (ii) and/or a conductive material (iii), and optionally an organic solvent,
b) optionally applying the mixture to at least one surface of a current collector, and
c) removing solvent.

If an organic solvent is used in step a), it is preferably selected from N-methylpyrrolidone (NMP) and isopropanol.

In one preferred embodiment, the method comprises preparing a mixture comprising the electrode active material, the binder and optionally the conductive material, blending this mixture, forming a sheet from the mixture and pressing this sheet onto a current collector formed from a metal foil or other materials as described above.

In another preferred embodiment, the electrode is prepared by adding the binder and optionally the conductive material to the electrode active material, dispersing the mixture in an organic solvent to prepare a paste for forming a mixed electrode layer, applying the paste onto at least one surface, e.g., one surface or both surfaces, of a current collector formed from a metal foil or other materials, drying the applied paste to form a mixed electrode layer, and optionally subjecting the layer to a processing step. In this embodiment of the method, the binder may also be dissolved or dispersed in an organic solvent before adding it to the electrode active material.

In another preferred embodiment, the electrode is prepared by adding the conductive material and optionally a binder to the electrode active material, dispersing the mixture in an organic solvent and filtering the dispersion to obtain a self-standing electrode layer. Optionally, the self-standing electrode may further be pressed.

In a further aspect, the present invention relates to the use of an electrode of the invention as a cathode in an aqueous or non-aqueous secondary battery.

Thus, the present invention also relates to a secondary battery comprising an electrode of the invention as cathode, an anode, an electrolyte, and a separator. The secondary battery of the invention may be aqueous or non-aqueous. Preferably, the anode comprises at least one of Li, Zn, Mg, Al, K, and Na.

Preferably, the cathode and anode of the secondary battery of the invention are laminated with an interjacent separator between them to form laminated electrodes. Optionally, the laminated electrodes are further wound into a spiral shape to form spiral-wound electrodes.

The separator preferably has sufficient strength and can retain as much electrolyte as possible. Thus, the separator is preferably a microporous film, a non-woven fabric, a filter, etc., that has a preferred thickness of 10 µm to 300 µm and a preferred porosity of 30 to 70%.

In one preferred embodiment, the secondary battery of the invention is a non-aqueous secondary battery, wherein the electrolyte is non-aqueous. In this case, the anode preferably comprises at least one of Li, Zn, Mg, Ca, Al, K, and Na. Moreover, the separator of the non-aqueous secondary battery preferably comprises at least one of polyethylene, polypropylene, an ethylene-propylene copolymer, glass, and combinations thereof.

One preferred embodiment of the non-aqueous secondary battery of the invention is a lithium-ion secondary battery. The non-aqueous secondary battery of the invention may also be a lithium secondary battery comprising metallic lithium in the anode.

In one embodiment of the non-aqueous secondary battery of the invention, the electrolyte is a solution electrolyte, preferably organic, more preferably a solution of LiTFSI or LiPF6 dissolved in an organic solvent, wherein the organic solvent is preferably selected from dimethoxyethane, dioxolane, ethylene carbonate, dimethyl carbonate, diethyl carbonate and combinations thereof.

Alternatively, the electrolyte of the non-aqueous secondary battery may be a solid electrolyte, preferably an inorganic solid electrolyte, more preferably a Li2S-P2S5-based electrolyte or a Li2S-GeS2-P2S5-based electrolyte. A lithium-ion secondary battery of the invention with a solid electrolyte may also be called an all-solid-state lithium-ion secondary battery.

In another preferred embodiment, the secondary battery of the invention is an aqueous secondary battery, wherein the electrolyte is aqueous. In this case, the anode preferably comprises at least one of Zn, Mg, and Al. The separator of the aqueous secondary battery preferably comprises at least one of cellulose, hydrophilic PTFE and glass.

The electrolyte of the aqueous secondary battery of the invention is preferably a solution prepared by dissolving a zinc salt, preferably ZnSO4 or Zn(OTf)2, in water or a combination of water and at least one of acetonitrile, ethylene glycol, and sulfolane.

Thus, one preferred embodiment of the aqueous secondary battery of the invention is a zinc-ion secondary battery, preferably an aqueous zinc-ion secondary battery comprising an aqueous electrolyte. The aqueous secondary battery of the invention may also be a zinc secondary battery comprising metallic zinc in the anode.

It has surprisingly been found that both non-aqueous and aqueous secondary batteries of the present invention display advantageous characteristics, including high stability and favorable energy densities. Surprisingly, in aqueous electrolytes, compounds of the invention show up to 88% capacity retention after 80 cycles at a current density of 100 mAg⁻¹ and energy densities of up to 191 Whkg⁻¹. At the same time, in non-aqueous electrolytes, compounds of the invention show up to 96 % capacity retention after 190 cycles at a current density of 50 mAg⁻¹ and energy densities of up to 931 Whkg⁻¹.

### Brief description of the drawings

- **Fig. 1**: Galvanostatic charge/discharge curves (left) and cycling stability (right) of a non-aqueous secondary battery as described in Example 4, comprising the compound of Example 1 as cathode active material.
- **Fig. 2**: Galvanostatic charge/discharge curves (left) and cycling stability (right) of a non-aqueous secondary battery as described in Example 4, comprising the compound of Example 2 as cathode active material.
- **Fig. 3**: Galvanostatic charge/discharge curves (left) and cycling stability (right) of a non-aqueous secondary battery as described in Example 4, comprising the compound of Example 3 as cathode active material.
- **Fig. 4**: Galvanostatic charge/discharge curves (left) and cycling stability (right) of an aqueous secondary battery as described in Example 5, comprising the compound of Example 1 as cathode active material.
- **Fig. 5**: Galvanostatic charge/discharge curves (left) and cycling stability (right) of an aqueous secondary battery as described in Example 5, comprising the compound of Example 2 as cathode active material.
- **Fig. 6**: Galvanostatic charge/discharge curves (left) and cycling stability (right) of an aqueous secondary battery as described in Example 5, comprising the compound of Example 3 as cathode active material.

### Examples

The following examples shall describe and illustrate the present invention in more detail without limiting the invention in any way.

### Example 1

BQPC was synthesized through the following route. A mixture of sodium rhodizonate (0.50 g, 2.34 mmol) and 2,3-diaminoquinoxaline (0.82 g, 5.14 mmol) was added to 50 mL of deoxygenated glacial acetic acid and heated at 120 °C for 48 h under inert atmosphere. Upon cooling to room temperature, the solid was filtered and washed with glacial acetic acid, acetone, ethanol and water. The product was additionally purified using 24 h Soxhlet extraction with ethanol. Obtained product was dried at 80 °C overnight yielding BQPC as a black powder in 65 % yield (yield is based on the formation of pure BQPC (1)). The end product is a mixture of BQPC (1) and BQPC (2), which could not be further purified due to insolubility of the compounds. ¹H-¹³C CP-MAS (ppm): 181.4, 179.7, 176.9, 148.7, 138.3, 130.5, 123.9, 117.5, 115.7. Mass spectrum (MALDI, m/z): [BQPC (2) + H]⁺ calculated for C₂₂H₉N₈O₂, 417.3; found, 417.2.

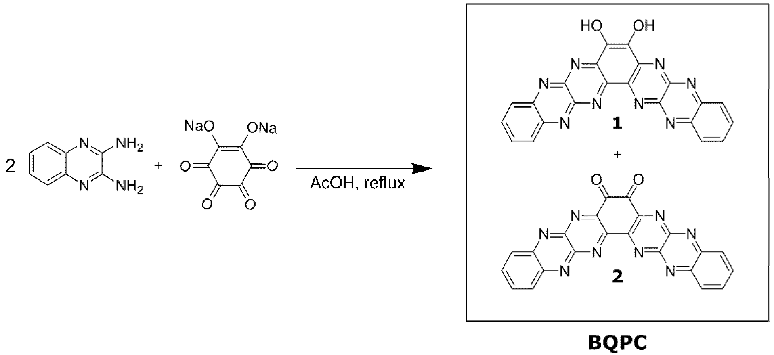

### Example 2

1,2,3,4,7,8,9,10,13,14,15,16-dodecamethoxydiquinoxalino[2,3-a:2',3'-c]phenazine (HATDB) was synthesized through the following route. 1,2,3,4-tetramethoxybenzene (2 g, 10.1 mmol) was slowly added in portions to 62 wt. % HNO₃ (185 mL) cooled using an ice bath. The mixture was left to stir for 1 h in an ice bath and for another 1 h at room temperature. During stirring evolution of brown nitrous oxides was observed. Afterwards the mixture was filtered and thoroughly washed with water to obtain the product in the form of light-yellow powder in 95 % yield. Next, the obtained product 1,2,3,4-tetramethoxybenzene-5,6-dinitrobenzene (2.0 g, 6.94 mmol) was slowly added to a stirred mixture of granular tin (4.77 g, 40.2 mmol) in 36 wt. % HCl (30 mL), and the mixture was heated at reflux for 2 h. Upon cooling to room temperature, the resulting crystalline solid was filtered. The solid was dissolved in water and the solution made alkaline using 4 M NaOH under nitrogen atmosphere. Afterwards the solution was extracted using chloroform and quickly filtered through short silica plug. Removal of the solvent under reduced pressure yielded product in the form of off-white crystals in 70 % yield.

Next, the obtained product 3,4,5,6-tetramethoxybenzene-1,2-diamine (S14) (1.0 g, 4.38 mmol) and hexaketocyclohexane octahydrate (HKC) (414 mg, 1.33 mmol) were added to deoxygenated glacial acetic acid (30 mL). The mixture was heated at 120 °C for 16 h. Upon cooling to room temperature the resulting solid was filtered, washed with ethanol, and dried at 80 °C overnight yielding the final product in the form of yellow powder in 70 % yield. Obtained product 1,2,3,4,7,8,9,10,13,14,15,16-dodecamethoxydiquinoxalino[2,3-a:2',3'-c]phenazine (HATDBP) (170 mg, 0.228 mmol) and pyridine hydrochloride (10 g, 45.6 mmol) were weighed in a glovebox into a heavy-walled glass pressure vessel. The mixture was heated at 180 °C for 2 h. Upon cooling to room temperature water was added to the mixture and the product was filtered and washed extensively with water, dichloromethane, and ethanol. The product was further purified with 24 h ethanol Soxhlet extraction. The final product (HATDB) was dried at 80 °C overnight yielding a dark brown powder in 95 % yield. ¹H-¹³C CP-MAS (ppm): 177.3, 144.4, 130.3. Mass spectrum (MALDI, m/z): [HATDB + H]⁺ calculated for C₂₄H₇N₆O₁₂, 571.0; found, 571.6.

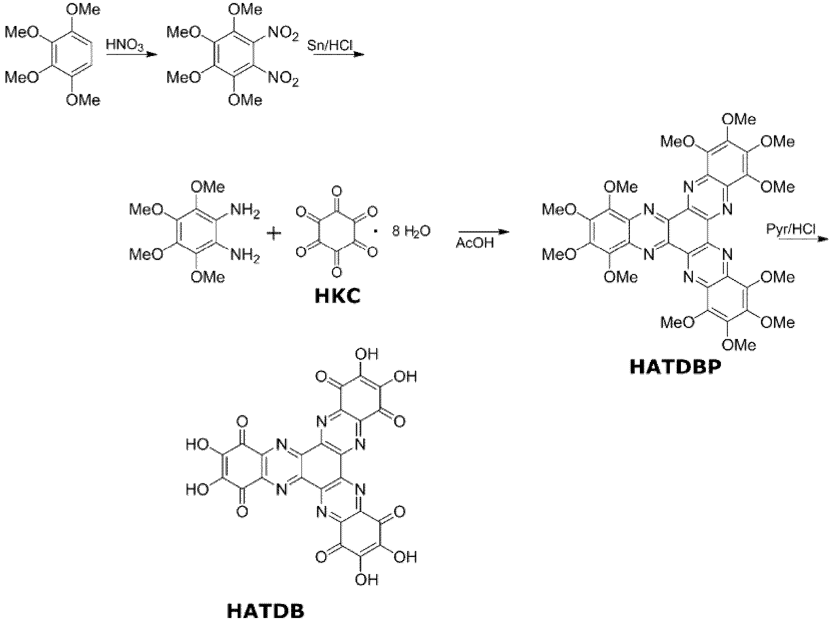

### Example 3

BBNPC (1), BBNPC (2), and BBNPC (3) were synthesized through the following route. A mixture of sodium rhodizonate (0.165 g, 0.771 mmol) and 2,3-diamino-1,4-dihydroxy-9,10-anthracenedione (0.479 g, 1.77 mmol) was added to 25 mL of deoxygenated glacial acetic acid and heated at 120 °C for 48 h under inert atmosphere. Upon cooling to room temperature, the solid was filtered and washed with glacial acetic acid, dimethyl formamide, acetone, ethanol and water. The product was additionally purified using 24 h Soxhlet extraction with ethanol. Obtained product was dried at 80 °C overnight yielding BBNPC as a black powder in 25 % yield (yield is based on the formation of pure BBNPC (1)). The end product is a mixture of BBNPC (1), BBNPC (2), and BBNPC (3).

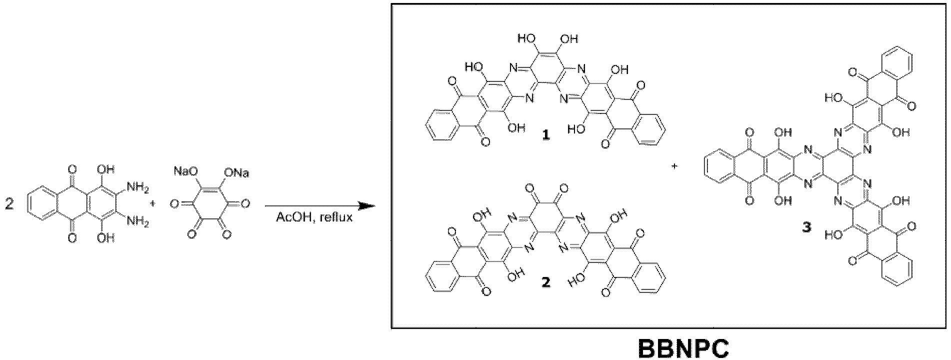

### Example 4: Evaluation of Electrodes (Half-Cells) in non-aqueous electrolytes

The compounds synthesized in Example 1, Example 2, and Example 3 (cathode active material) were mixed with Printex XE2 (conductive material) and PTFE (binder) in a weight ratio of 60 : 30 : 10 (cathode active material: conductive material: binder) and ball milled in a planetary ball mill at 300 rpm for 30 min in an ambient atmosphere. The obtained slurry was rolled into a film, pressed on an aluminum mesh (mesh size 100) current collector and cut into electrode discs (ϕ = 12 mm), which were afterwards dried at 50 °C for 1 day. Stainless steel Swagelok-type battery cells were assembled in an argon-filled glovebox (O₂ < 1 ppm, H₂O < 1 ppm) by separating working electrodes and lithium foil discs (ϕ = 12 mm) with 2 pieces (ϕ = 13 mm) of Celgard 2320 separators wetted with 3 drops of 1 M LiTFSI in 1:1 (v/v) dioxolane and dimethoxyethane. The batteries were characterized by measuring galvanostatic charge/discharge curves and cycling stabilities using methods well known to the person skilled in the art.

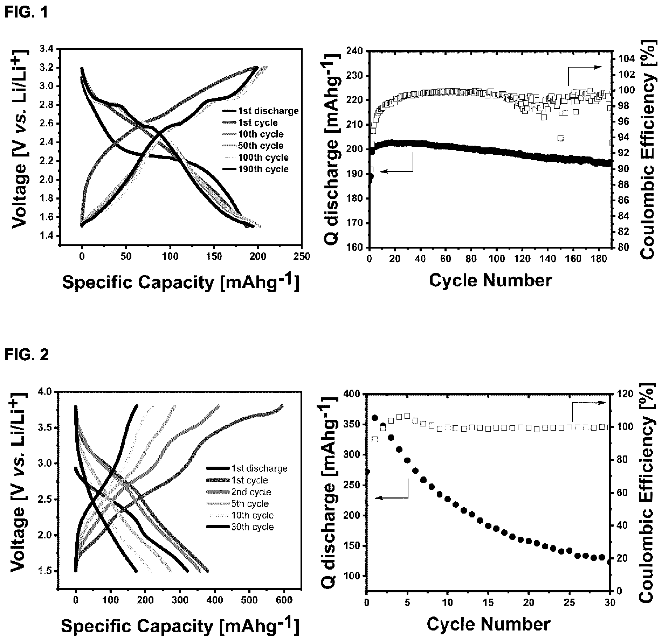

Galvanostatic charge/discharge curves and cycling stabilities of the non-aqueous secondary batteries comprising the compounds of Examples 1, 2 and 3 as cathode active materials are shown in Figures 1, 2 and 3, respectively.

For a cathode comprising the compound of Example 1, the discharge capacity was as high as 202 mAhg⁻¹ with an average voltage of 2.29 V vs. Li/Li⁺, and the capacity retention reached a value of 96 % after 190 cycles at a current density of 50 mAg⁻¹ (see Fig. 1).

For a cathode comprising the compound of Example 2, the discharge capacity was as high as 380 mAhg⁻¹ with an average voltage of 2.45 V vs. Li/Li⁺, and the capacity retention reached a value of 46 % after 30 cycles at a current density of 50 mAg⁻¹ (see Fig. 2).

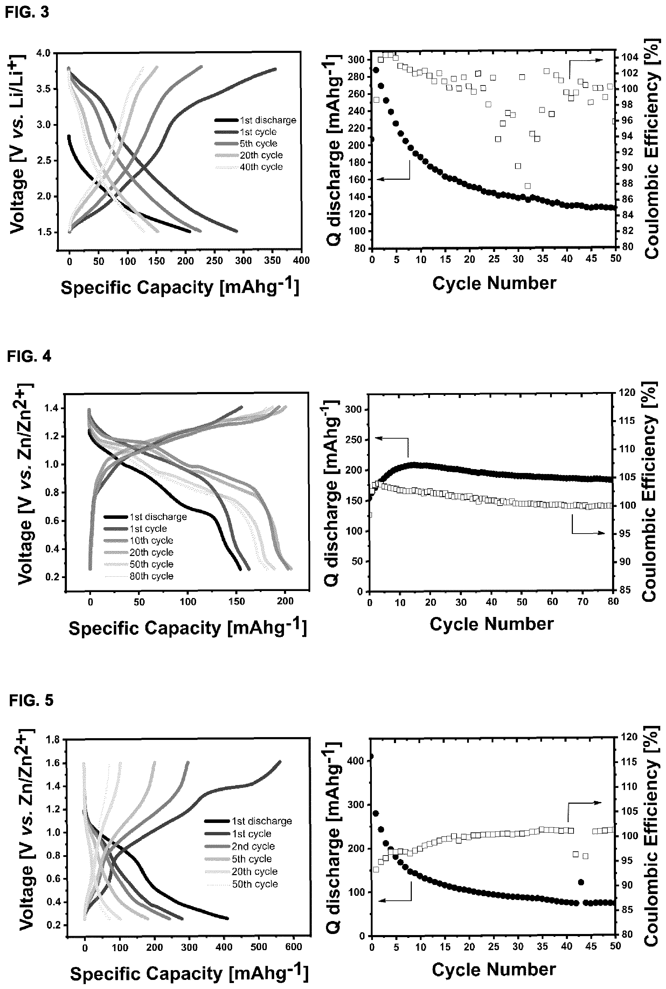

For a cathode comprising the compound of Example 3, the discharge capacity was as high as 284 mAhg⁻¹ with an average voltage of 2.43 V vs. Li/Li⁺, and the capacity retention reached a value of 43 % after 50 cycles at a current density of 50 mAg⁻¹ (see Fig. 3).

Thus, in non-aqueous electrolytes, compounds of the present invention show great stability of up to 96 % capacity retention after 190 cycles at a current density of 50 mAg⁻¹, as well as high energy densities of up to 931 Whkg⁻¹.

### Example 5: Evaluation of Electrodes (Half-Cells) in aqueous electrolytes

The compounds synthesized in Example 1, Example 2, and Example 3 (cathode active material) were mixed with Printex XE2 (conductive material) and PTFE (binder) in a weight ratio of 60 : 30 : 10 (cathode active material: conductive material: binder) and ball milled in a planetary ball mill at 300 rpm for 30 min in an ambient atmosphere. The obtained slurry was rolled into a film and cut into self-standing electrode discs (ϕ = 10 mm), which were afterward dried at 50 °C for 1 day. Stainless steel Swagelok-type battery cells were assembled in ambient atmosphere by separating working electrodes and zinc foil discs (ϕ = 12 mm) with 2 pieces (ϕ = 13 mm) of glass fiber separators (Whatman GF/A) wetted with 3 drops of 3 M ZnSO₄ in H₂O. The batteries were characterized by measuring galvanostatic charge/discharge curves and cycling stabilities using methods well known to the person skilled in the art.

Galvanostatic charge/discharge curves and cycling stabilities of the aqueous secondary batteries comprising the compounds of Examples 1, 2 and 3 as cathode active materials are shown in Figures 4, 5 and 6, respectively.

For a cathode comprising the compound of Example 1, the discharge capacity was as high as 208 mAhg⁻¹ with an average voltage of 0.92 V vs. Zn/Zn²⁺, and the capacity retention reached a value of 88 % after 80 cycles at a current density of 100 mAg⁻¹ (see Figure 4).

For a cathode comprising the compound of Example 2, the discharge capacity was as high as 410 mAhg⁻¹, and the capacity retention reached a value of 18 % after 50 cycles at a current density of 100 mAg⁻¹ (see Figure 5).

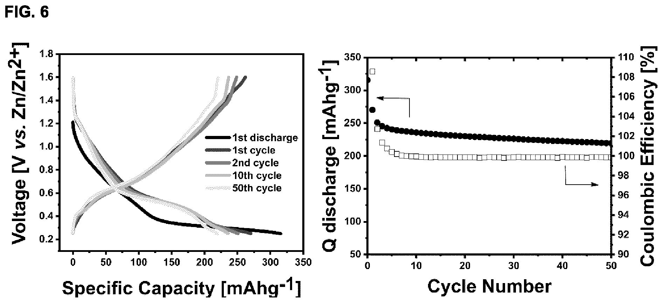

For a cathode comprising the compound of Example 3, the discharge capacity was as high as 250 mAhg⁻¹ with an average voltage of 0.64V vs. Zn/Zn²⁺, and the capacity retention reached a value of 91 % after 50 cycles at a current density of 100 mAg⁻¹ (see Figure 6).

Thus, in aqueous electrolytes, compounds of the present invention show great stability of up to 88 % capacity retention after 80 cycles at a current density of 100 mAg⁻¹, high energy density of up to 191 Whkg⁻¹, as well as favorable voltage profile characteristics.

The present invention is summarized by the following items:
1. A fused polycyclic compound represented by any one of the general formulas (I) to (III) 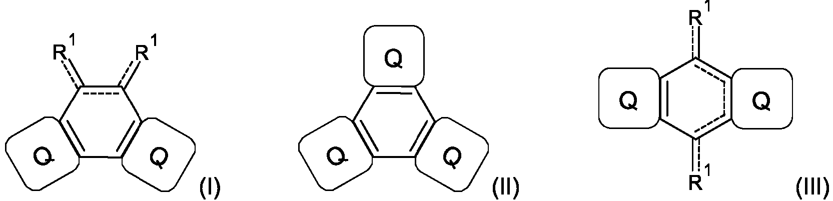 with Q being represented by the general formula (IV) 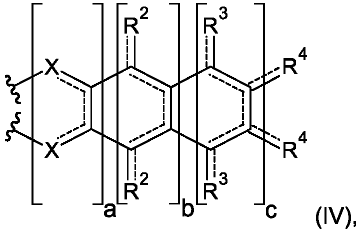 wherein
   - X: is 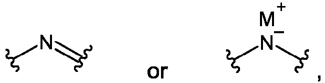
   - R¹: is 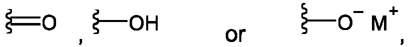
   - R²: is independently selected from or a linear or 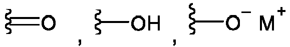 branched, preferably linear, alkoxy group with 1-6 C atoms, preferably with 1-3 C atoms,
   - R³: is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,
   - R⁴: is independently selected from a hydrogen 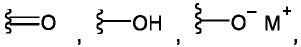 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
   - M⁺: represents cations independently selected from a group represented by protons, Li⁺, Na⁺, K⁺, ½ Mg²⁺, ½ Ca²⁺ and ½ Zn²⁺,
   - a: is an integer of 1-2,
   - b: is an integer of 0-2, and an integer of 1-2 if the fused polycyclic compound corresponds to formula (II),
   - c: is an integer of 0-1,

   wherein the sum of integers a, b and c is 2-4, and
   bonds that are represented by a solid line and a dashed line indicate a single bond or a double bond.
2. A fused polycyclic compound according to item 1, wherein Q is represented by formula (IVa) 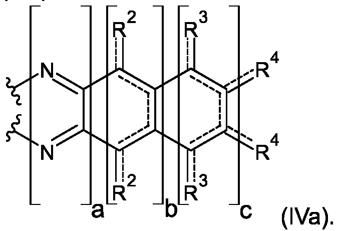
3. A fused polycyclic compound according to items 1 or 2, wherein the fused polycyclic compound is represented by general formula (I), and wherein
   a = 2, b = 0 and c = 1, or
   a = 1, b = 1 and c = 0, or
   a = 1, b = 2 and c = 1, or
   a = 2, b = 0 and c = 0,
   preferably a = 2, b = 0 and c = 1 or a = 1, b = 2 and c = 1.
4. A fused polycyclic compound according to items 1 or 2, wherein the fused polycyclic compound is represented by general formula (II), and wherein
   a = 1, b = 1 and c = 0, or
   a = 1, b = 2 and c = 1,
   preferably a = 1, b = 2 and c = 1.
5. A fused polycyclic compound according to items 1 or 2, wherein the fused polycyclic compound is represented by general formula (III), and wherein
   a = 2, b = 0 and c = 1, or
   a = 1, b = 1 and c = 0, or
   a = 1, b = 2 and c = 1, or
   a = 2, b = 0 and c = 0.
6. A fused polycyclic compound according to any one of the preceding items, wherein the fused polycyclic compound is represented by any one of the following formulas: 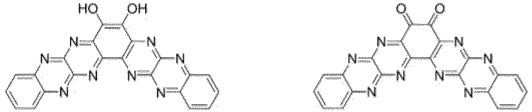 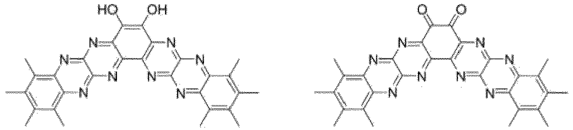 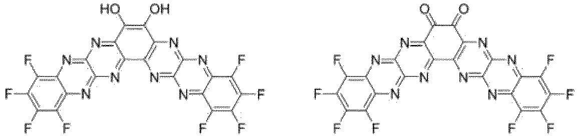 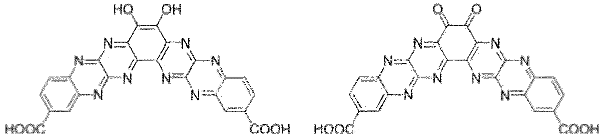 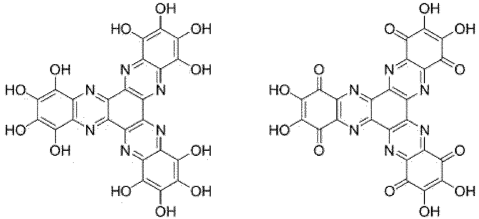 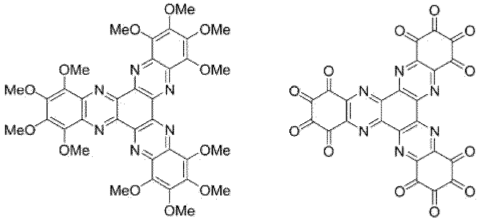 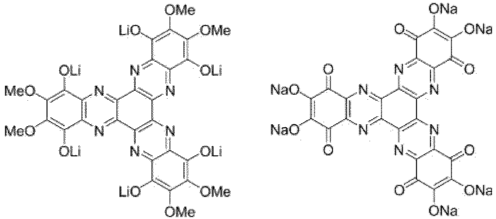 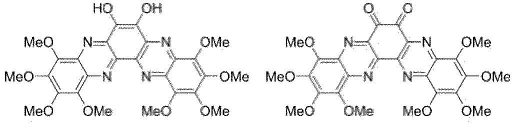 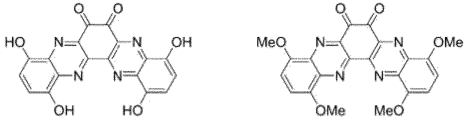 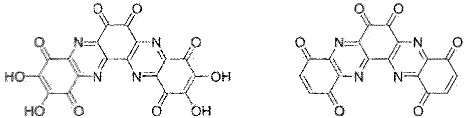 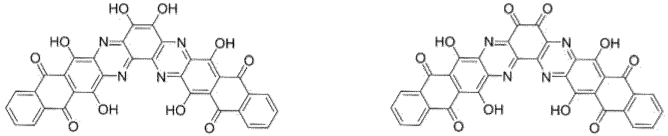 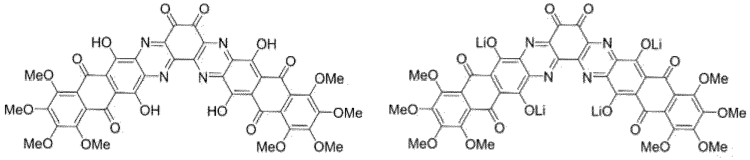 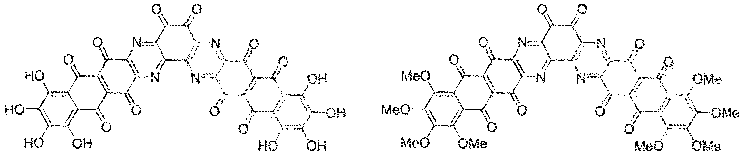 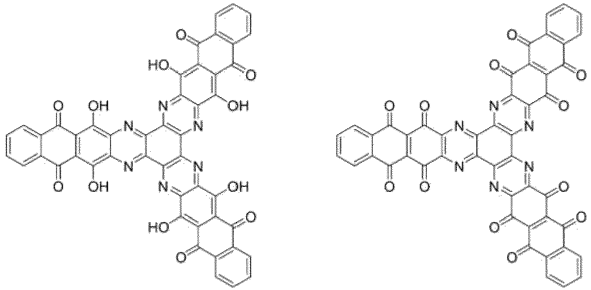 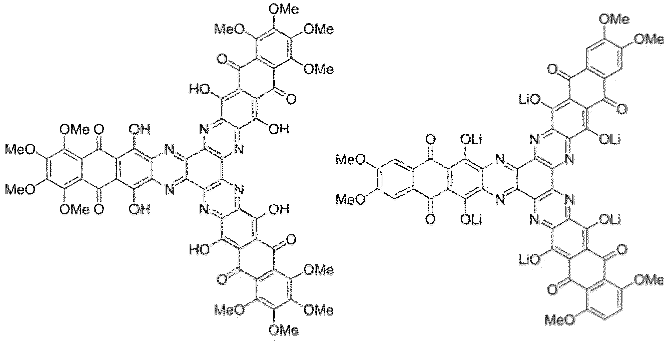 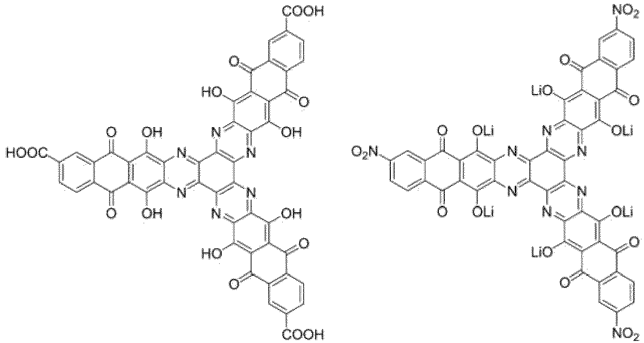 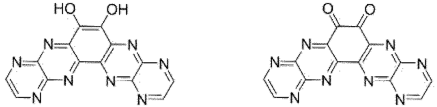 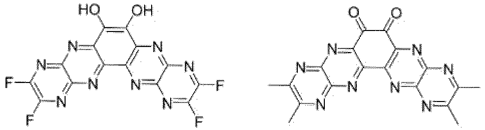 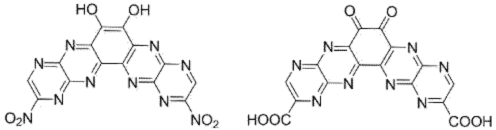 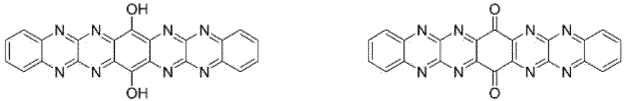 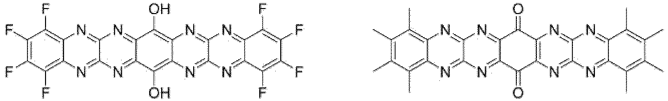 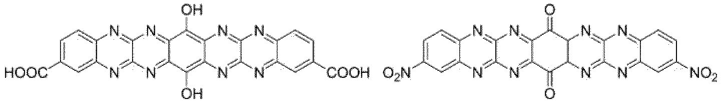 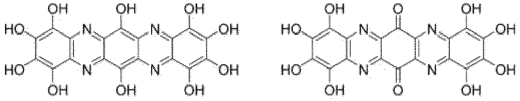 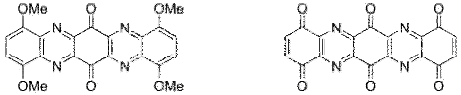 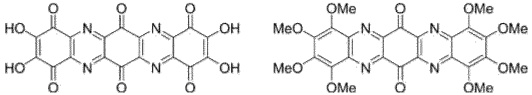 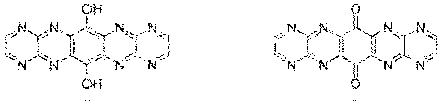 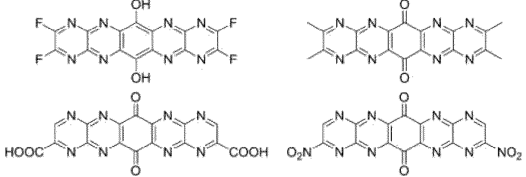 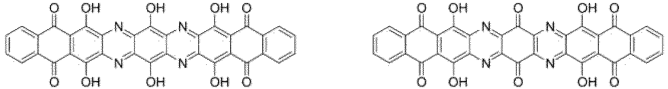 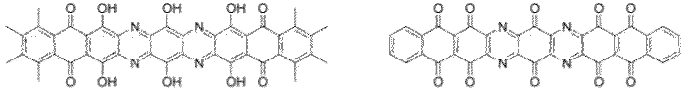 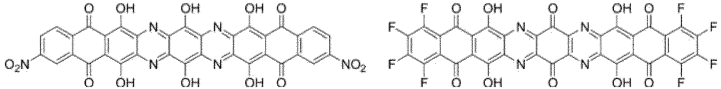
7. A fused polycyclic compound according to any one of the preceding items, wherein the fused polycyclic compound is represented by any one of the following formulas: 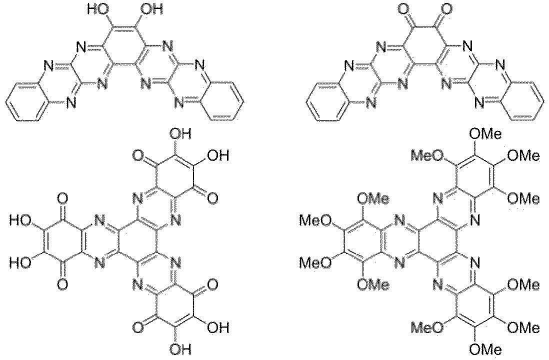 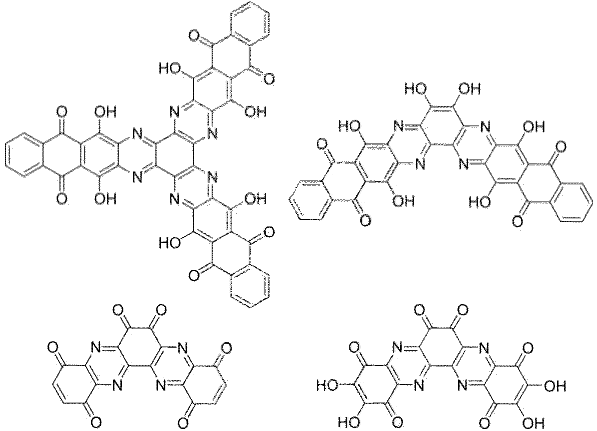
8. A method of preparing a compound according to any one of the preceding items, wherein the method comprises reacting a compound of the general formula (V) 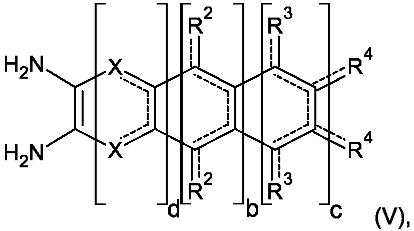 wherein
   - X: is 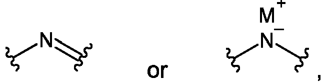
   - R²: is independently selected from or a linear or branched, preferably linear, alkoxy group with 1-6 C atoms, preferably with 1-3 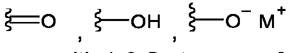 C atoms,
   - R³: is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,

   - R⁴: is independently selected from a hydrogen 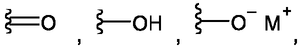 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
   - M⁺: represents cations independently selected from a group represented by protons, Li⁺, Na⁺, K⁺, ½ Mg²⁺, ½ Ca²⁺ and ½ Zn²⁺,
   - b: is an integer of 0-2, and an integer of 1-2 if the fused polycyclic compound corresponds to formula (II),
   - c: is an integer of 0-1,
   - d: is an integer of 0-1,

   wherein the sum of integers b, c and d is 1-3, and
   bonds that are represented by a solid line and a dashed line indicate a single bond or a double bond,
   with a compound selected from 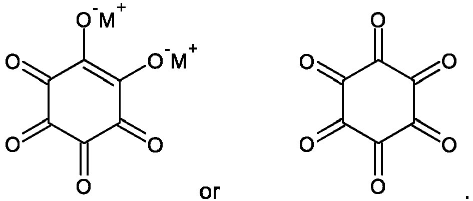
9. A method according to item 8, wherein the reaction is carried out in a solvent, preferably a deoxygenated solvent, wherein the solvent is preferably selected from glacial acetic acid, Methanol, ethanol, water, and mixtures thereof.
10. A method according to any one of items 8-9, wherein a catalyst is added to the reaction, wherein the catalyst is preferably selected from 2-iodoxybenzoic acid, Zirconium(IV) chloride, sulfamic acid and ammonium chloride.
11. A method according to any one of items 8-10, wherein the reaction is conducted at a temperature of 110-130°C, preferably of 120°C,
   preferably for 8-72 h, more preferably for 16-48 h.
12. A method according to any one of items 8-11, wherein one or more of substituents R², R³ and R⁴ in formula (V) are protected by protecting groups, and wherein the method further comprises a step of deprotecting said substituents with a deprotecting agent.
13. A method according to any one of items 8-12, further comprising an oxidation or reduction step.
14. A method according to any one of items 8-13, wherein a product of the reaction is filtered and washed with a solvent, preferably with glacial acetic acid, acetone, ethanol, water, dichloromethane and/or dimethyl formamide.
15. A method according to the preceding item, wherein the product of the reaction is additionally purified by Soxhlet extraction with ethanol.
16. A method according to any one of items 8-15, wherein the product of the reaction is further dried at a temperature of 70-90°C, preferably of 80°C, for 3-24 hours, preferably overnight.
17. Use of a fused polycyclic compound according to any one of items 1-7 as an electrode active material for an aqueous or non-aqueous secondary battery.
18. Use according to item 17 as a cathode active material.
19. An electrode for an aqueous and/or non-aqueous secondary battery, comprising
   (i) an electrode active material comprising at least one fused polycyclic compound according to any one of items 1-7,
      and further comprising at least one of
   (ii) a binder, and/or
   (iii) a conductive material,
   wherein the electrode preferably comprises the electrode active material (i), the binder (ii) and the conductive material (iii).
20. An electrode according to item 19, wherein the electrode active material (i) is present in an amount of 40-95 wt.%, preferably 50-80 wt.%, with respect to the total weight of the electrode.
21. An electrode according to any one of items 19-20, wherein the binder (ii) is selected from polyvinylidene fluoride (PVDF), polytetrafluoroethylene, polyacrylic acid, sodium carboxymethyl cellulose, styrene butadiene rubber, and combinations thereof.
22. An electrode according to any one of items 19-21, wherein the binder (ii) is present in an amount of 2-20 wt.%, preferably 5-15 wt.%, with respect to the total weight of the electrode.
23. An electrode according to any one of items 19-22, wherein the conductive material (iii) is a carbon-based conductive material, preferably selected from graphite,
   carbon black, such as acetylene black, Ketjenblack, or Printex XE2,
   amorphous carbon materials, such as carbon materials prepared by forming amorphous carbon on the surface,
   fibrous carbon, such as vapor-grown carbon fiber and carbon fiber prepared by carbonization after spinning of pitch,
   carbon nanotubes, such as multi-walled or single-walled carbon nanotubes,
   and combinations thereof.
24. An electrode according to any one of items 19-23, wherein the conductive material (iii) is present in an amount of 2-30 wt.%, preferably 5-20 wt.%, with respect to the total weight of the electrode.
25. An electrode according to any one of items 19-24, which is formed on at least one surface of a current collector.
26. An electrode according to item 25, wherein the current collector is a foil, mesh, perforated metal or expanded metal.
27. An electrode according to any one of items 25-26, wherein the current collector is made of a metal or metal alloy, preferably of aluminum, stainless steel, nickel, titanium or alloys thereof.
28. An electrode according to any one of items 25-27, wherein the thickness of the electrode formed on at least one surface of a current collector is in the range of 1 µm to 200 µm.
29. A method of preparing an electrode according to any one of items 19-28, comprising the steps:
   a) preparing a mixture comprising the electrode active material (i), at least one of a binder (ii) and/or a conductive material (iii), and optionally an organic solvent,
   b) optionally applying the mixture to at least one surface of a current collector, and
   c) removing solvent.
30. The method of item 29, wherein the organic solvent is selected from N-methylpyrrolidone (NMP) and isopropanol.
31. The method of item 29, wherein step (a) comprises blending the mixture, e.g., by ball milling.
32. The method of any one of items 29 or 31, wherein a sheet or film formed from the mixture prepared in step (a) is pressed onto at least one surface of a current collector in step (b).
33. The method of any one of items 29-30, wherein step (a) comprises preparing a paste by dispersing the mixture in the organic solvent, and step (b) comprises applying the paste to at least one surface of a current collector.
34. The method of any one of items 29-30, wherein the mixture dispersed in an organic solvent is filtered and optionally pressed to obtain a self-standing electrode.
35. Use of an electrode according to any one of items 19-28 as a cathode in an aqueous or non-aqueous secondary battery.
36. A secondary battery comprising an electrode according to any one of items 19-28 as cathode, an anode, an electrolyte, and a separator.
37. The secondary battery of item 36, wherein the cathode and anode are laminated with an interjacent separator between them to form laminated electrodes, optionally wherein the laminated electrodes are further wound into a spiral shape to form spiral-wound electrodes.
38. The secondary battery of any one of items 36-37, wherein the anode comprises at least one of Li, Zn, Mg, Al, K, and Na.
39. The secondary battery of any one of items 36-38, wherein the separator is a microporous film, a non-woven fabric or a filter.
40. The secondary battery of any one of items 36-39, wherein the separator has a thickness of 10 µm to 300 µm.
41. The secondary battery of any one of items 36-40, wherein the separator has a porosity of 30 to 70%.
42. The secondary battery of any one of items 36-41, which is a non-aqueous secondary battery, wherein the electrolyte is non-aqueous.
43. The non-aqueous secondary battery of item 42, wherein the electrolyte is a solution electrolyte, preferably organic, more preferably a solution of LiTFSI or LiPF₆ dissolved in an organic solvent, wherein the organic solvent is preferably selected from dimethoxyethane, dioxolane, ethylene carbonate, dimethyl carbonate, diethyl carbonate and combinations thereof.
44. The non-aqueous secondary battery of item 42, wherein the electrolyte is a solid electrolyte, preferably an inorganic solid electrolyte, more preferably a Li₂S-P₂S₅-based electrolyte or a Li₂S-GeS₂-P₂S₅-based electrolyte.
45. The non-aqueous secondary battery of any one of items 42-44, wherein the anode comprises at least one of Li, Zn, Mg, Ca, Al, K, and Na.
46. The non-aqueous secondary battery of any one of items 42-45, wherein the separator comprises at least one of polyethylene, polypropylene, an ethylene-propylene copolymer, glass, and combinations thereof.
47. The secondary battery of any one of items 36-41, which is an aqueous secondary battery, wherein the electrolyte is aqueous.
48. The aqueous secondary battery of item 47, wherein the electrolyte is a solution prepared by dissolving a zinc salt, preferably ZnSO₄ or Zn(OTf)₂, in water or a combination of water and at least one acetonitrile, ethylene glycol, and sulfolane.
49. The aqueous secondary battery of any one of items 47-48, wherein the anode comprises at least one of Zn, Mg, and Al.
50. The aqueous secondary battery of any one of items 47-49, wherein the separator comprises at least one of cellulose, hydrophilic PTFE and glass.

## Claims

1. A fused polycyclic compound represented by any one of the general formulas (I) to (III) 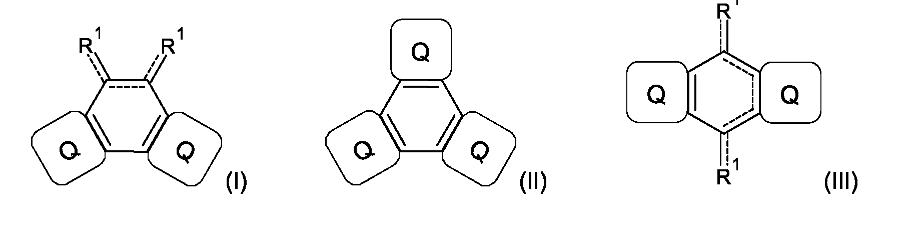 with Q being represented by the general formula (IV) 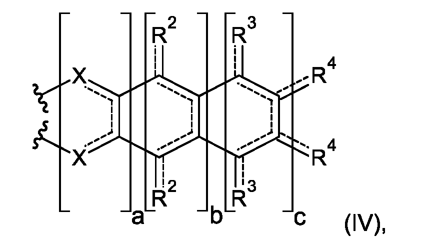 wherein
X is 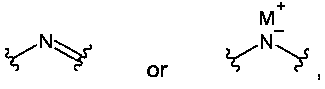
R¹ is 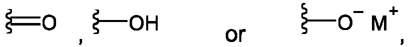
R² is independently selected from or a linear or 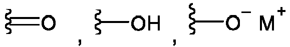 branched, preferably linear, alkoxy group with 1-6 C atoms, preferably with 1-3 C atoms,
R³ is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,
R⁴ is independently selected from a hydrogen 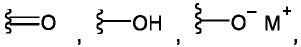 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
M⁺ represents cations independently selected from a group represented by protons, Li⁺, Na⁺, K⁺, ½ Mg²⁺, ½ Ca²⁺ and ½ Zn²⁺,
a is an integer of 1-2,
b is an integer of 0-2, and an integer of 1-2 if the fused polycyclic compound corresponds to formula (II),
c is an integer of 0-1,
wherein the sum of integers a, b and c is 2-4, and
bonds that are represented by a solid line and a dashed line indicate a single bond or a double bond.

2. A fused polycyclic compound according to claim 1, wherein Q is represented by formula (IVa)

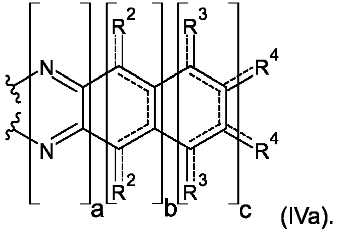

3. A fused polycyclic compound according to claims 1 or 2, wherein the fused polycyclic compound is represented by general formula (I), and wherein
a = 2, b = 0 and c = 1, or
a = 1, b = 1 and c = 0, or
a = 1, b = 2 and c = 1, or
a = 2, b = 0 and c = 0,
preferably a = 2, b = 0 and c = 1 or a = 1, b = 2 and c = 1.

4. A fused polycyclic compound according to claims 1 or 2, wherein the fused polycyclic compound is represented by general formula (II), and wherein
a = 1, b = 1 and c = 0, or
a = 1, b = 2 and c = 1,
preferably a = 1, b = 2 and c = 1.

5. A fused polycyclic compound according to claims 1 or 2, wherein the fused polycyclic compound is represented by general formula (III), and wherein
a = 2, b = 0 and c = 1, or
a = 1, b = 1 and c = 0, or
a = 1, b = 2 and c = 1, or
a = 2, b = 0 and c = 0.

6. A fused polycyclic compound according to any one of the preceding claims, wherein the fused polycyclic compound is represented by any one of the following formulas: 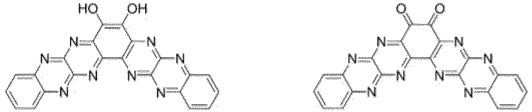 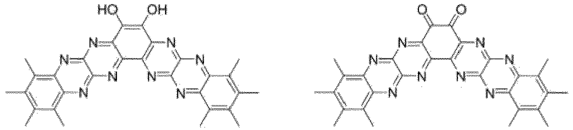 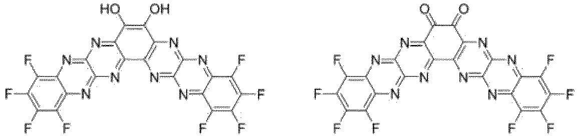 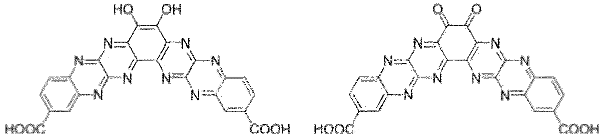 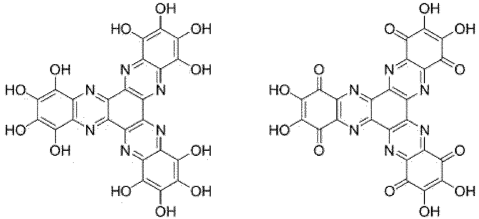 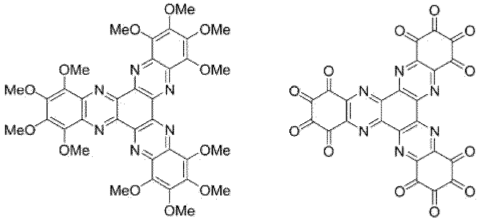 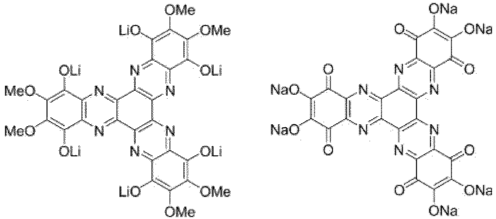 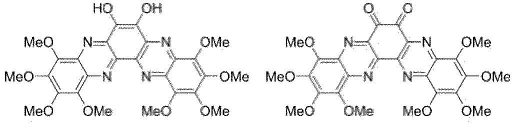 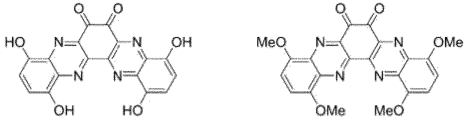 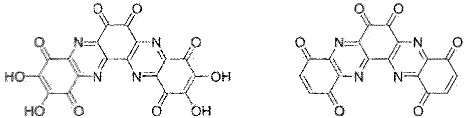 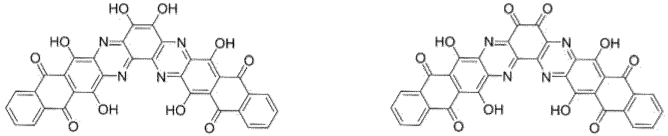 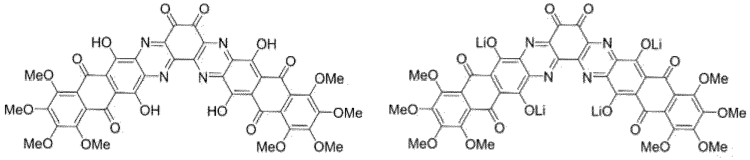 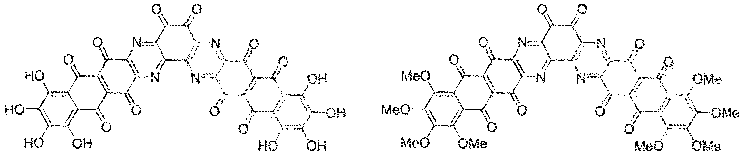 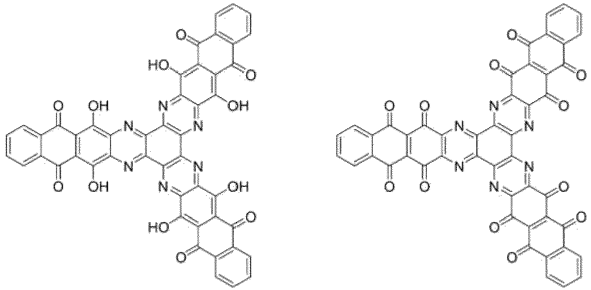 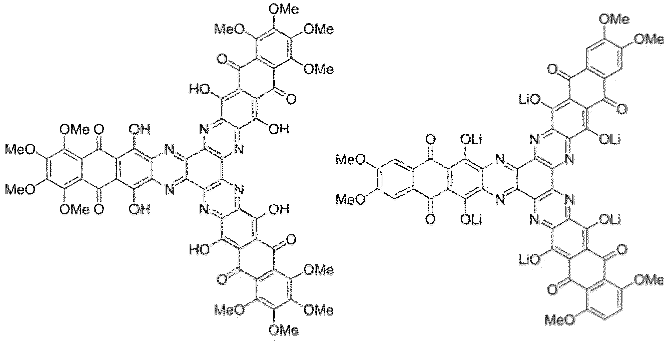 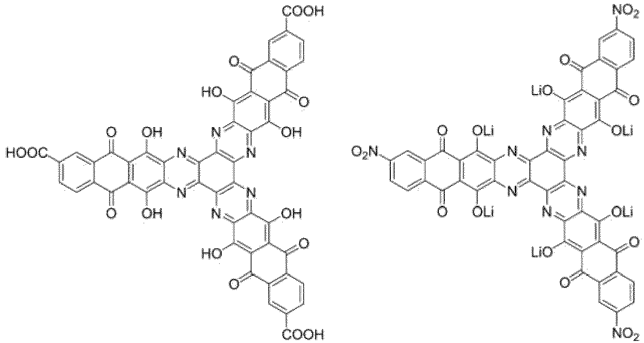 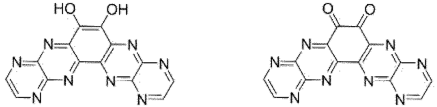 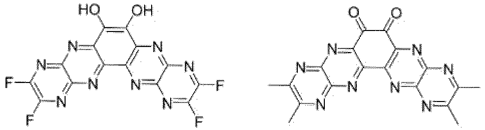 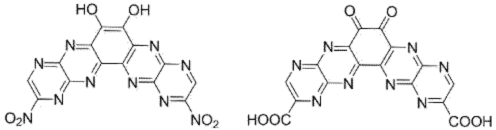 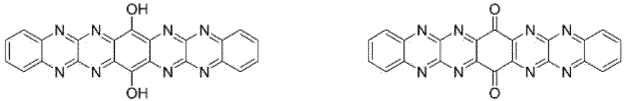 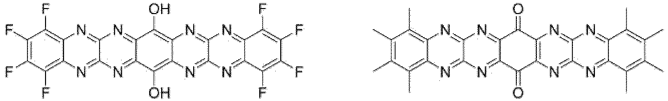 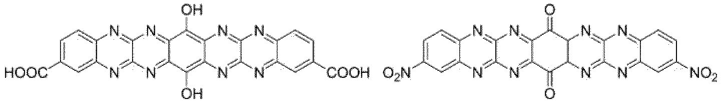 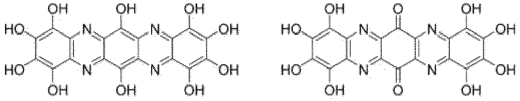 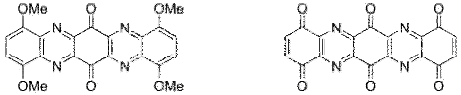 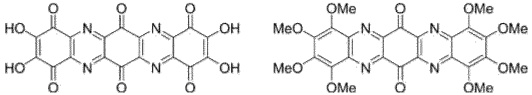 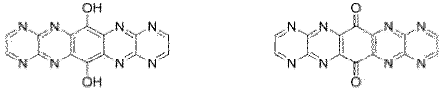 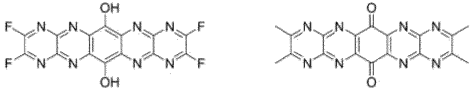 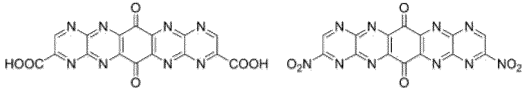 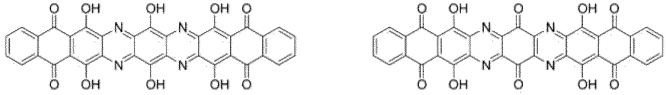 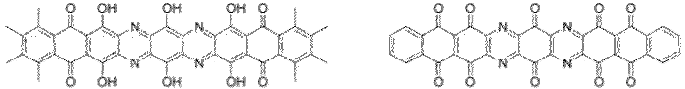 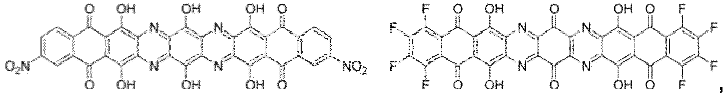 wherein the fused polycyclic compound is preferably represented by any one of the following formulas:

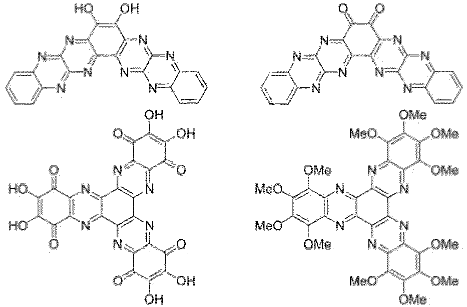

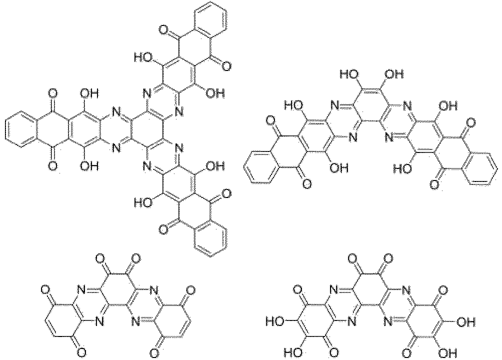

7. A method of preparing a compound according to any one of the preceding claims, wherein the method comprises reacting a compound of the general formula (V) 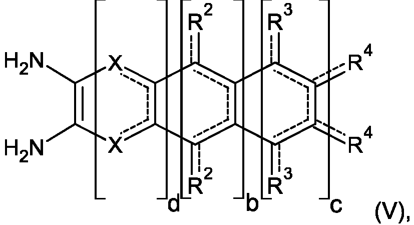 wherein
X is 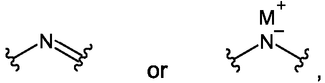
R² is independently selected from or a linear or 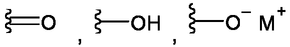 branched, preferably linear, alkoxy group with 1-6 C atoms, preferably with 1-3 C atoms,
R³ is independently selected from a hydrogen atom, halogen atom or an organic group, wherein the organic group is preferably selected from a C₁₋₆-alkyl, C₁₋₆-alkoxy, aryl, cyano, nitro, hydroxy or C₁₋₆-carboxy group,
R⁴ is independently selected from a hydrogen 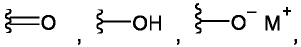 atom, halogen atom or an organic group, wherein the organic group is preferably selected from a C₁₋₆-alkyl, C₁₋₆-alkoxy, aryl, cyano, nitro, hydroxy or C₁₋₆-carboxy group,
M⁺ represents cations independently selected from a group represented by protons, Li⁺, Na⁺, K⁺, ½ Mg²⁺, ½ Ca²⁺ and ½ Zn²⁺,
b is an integer of 0-2, and an integer of 1-2 if the fused polycyclic compound corresponds to formula (II),
c is an integer of 0-1,
d is an integer of 0-1,
wherein the sum of integers b, c and d is 1-3, and
bonds that are represented by a solid line and a dashed line indicate a single bond or a double bond,
with a compound selected from

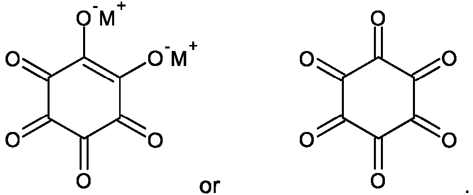

8. Use of a fused polycyclic compound according to any one of claims 1-6 as an electrode active material for an aqueous or non-aqueous secondary battery, preferably as a cathode active material for an aqueous or non-aqueous secondary battery.

9. An electrode for an aqueous and/or non-aqueous secondary battery, comprising
(i) an electrode active material comprising at least one fused polycyclic compound according to any one of claims 1-6,
and further comprising at least one of
(ii) a binder, and/or
(iii) a conductive material, preferably a carbon-based conductive material,
wherein the electrode preferably comprises the electrode active material (i), the binder (ii) and the conductive material (iii).

10. An electrode according to claim 9, wherein the electrode active material (i) is present in an amount of 40-95 wt.%, preferably 50-80 wt.%, with respect to the total weight of the electrode, and/or
wherein the binder (ii) is present in an amount of 2-20 wt.%, preferably 5-15 wt.%, with respect to the total weight of the electrode, and/or
wherein the conductive material (iii) is present in an amount of 2-30 wt.%, preferably 5-20 wt.%, with respect to the total weight of the electrode.

11. An electrode according to any one of claims 9-10, wherein the binder (ii) is selected from polyvinylidene fluoride (PVDF), polytetrafluoroethylene, polyacrylic acid, sodium carboxymethyl cellulose, styrene butadiene rubber, and combinations thereof.

12. An electrode according to any one of claims 9-11, which is formed on at least one surface of a current collector,
wherein the current collector is preferably a foil, mesh, perforated metal or expanded metal and/or is preferably made of a metal or metal alloy, preferably of aluminum, stainless steel, nickel, titanium or alloys thereof.

13. A method of preparing an electrode according to any one of claims 9-12, comprising the steps:
a) preparing a mixture comprising the electrode active material (i), at least one of a binder (ii) and/or a conductive material (iii), and optionally an organic solvent,
b) optionally applying the mixture to at least one surface of a current collector, and
c) removing solvent.

14. Use of an electrode according to any one of claims 9-12 as a cathode in an aqueous or non-aqueous secondary battery.

15. An aqueous or non-aqueous secondary battery comprising an electrode according to any one of claims 9-12 as cathode, an anode, an electrolyte, and a separator.
